**Europäisches Patentamt**

**European Patent Office**

**Office européen des brevets**

(19)

(11) Veröffentlichungsnummer: **0 090 289**
**B1**

# (12) EUROPÄISCHE PATENTSCHRIFT

(45) Veröffentlichungstag der Patentschrift:
26.02.86

(51) Int. Cl.⁴: **A 61 L 15/07**

(21) Anmeldenummer: **83102685.1**

(22) Anmeldetag: **18.03.83**

(54) **Feuchtigkeitshärtende Polyurethanstützverbände.**

(30) Priorität: **30.03.82 DE 3211634**

(43) Veröffentlichungstag der Anmeldung:
**05.10.83 Patentblatt 83/40**

(45) Bekanntmachung des Hinweises auf die Patenterteilung:
**26.02.86 Patentblatt 86/9**

(84) Benannte Vertragsstaaten:
**AT BE CH DE FR GB IT LI LU NL SE**

(56) Entgegenhaltungen:
**WO - A - 81/00671**
**FR - A - 2 332 001**

(73) Patentinhaber: **BAYER AG, Konzernverwaltung RP**
**Patentabteilung, D-5090 Leverkusen 1 Bayerwerk (DE)**

(72) Erfinder: **Wegner, Christian, Dr., Roggendorf**
**Strasse 65E, D-5000 Köln 80 (DE)**
Erfinder: **Schneider, Gottfried, Dr., Paul-Klee-Strasse 62,**
**D-5090 Leverkusen 1 (DE)**
Erfinder: **Mayer, Wolfram, Dr., Wagnerstrasse 18,**
**D-5060 Bergisch-Gladbach (DE)**

0 090 289

**Beschreibung**

Die vorliegende Erfindung betrifft ein Verbandmaterial zur Herstellung von Stützverbänden, bestehend aus einem Raschelgewirke aus Natur- und/oder Kunststoffaser als Trägermaterial, welches mit einem feuchtigkeitshärtenden, Isocyanatgruppen aufweisenden Harz beschichtet und/oder imprägniert ist.

Feuchtigkeitshärtende Polyurethanstützverbände werden z. B. in den deutschen Auslegeschriften 2 357 931 und 2 651 089 beschrieben. Es handelt sich hierbei um flexible Flächengebilde, zumeist in Bandform, als Trägermaterial, die mit einem Isocyanatgruppen aufweisenden imprägnierungsmittel getränkt sind, welches durch Reaktion mit Wasser aushärtet. Diese Verbandmaterialien können in derselben Weise appliziert werden wie konventionelle Gipsbinden, d. h. die Binde wird in Wasser getaucht, gewickelt und härtet anschließend zu einem starren Verband aus.

Gegenüber Gipsverbänden weisen solche Polyurethanstützverbände insbesondere folgende Vorteile auf: Geringes Gewicht und Volumen, Wasserunempfindlichkeit, Porosität und, im Falle von textilem Trägermaterial, hervorragende Röntgentransparenz. Ein handelsübliches Produkt dieser Art ist der Stützverband Baycast R der Fa. Bayer AG, bei dem als flexibles Trägermaterial ein Leinengewebe aus Baumwolle verwendet wird. Dieses Gewebe weist ein Flächengewicht von ca. 60 g/ m² auf, bei einer Fadenzahl von durchschnittlich 11 Fäden/ cm in Kettrichtung und 7 Fäden/cm im Schußrichtung. Das Verhältnis des gemittelten Abstandes zweier Kettfäden zu gemitteltem Abstand zweier Schußfäden beträgt somit etwa 0,6. Binden mit einem Trägermaterial aus Leinengewebe ergeben aufgrund ihrer festen und relativ dichten Webstruktur Stützverbände mit guter Festigkeit und glatter Oberfläche. Noch nicht voll befriedigend ist bei Verwendung von Leinengewebe als Trägermaterial jedoch der Anlegekomfort, in folge mangelnder Dehnbarkeit des Gewebes. Darüber hinaus bewirkt die dichte und glatte Oberfläche des ausgehärteten Stützverbandes eine verminderte Porosität.

Es hat daher nicht an Versuchen gefehlt, verbesserte Trägermaterialien für Polyurethanstützverbände bereitzustellen. Ein solcher Verband mit verbessertem Anlegekomfort auf textiler Basis ist beispielsweise CutterCast R der Firma Cutter Laboratories, Inc., Berkeley, USA, bei dem als Trägermaterial ein Raschelgewirke aus Baumwolle und Polyester eingesetzt wird. Dieses Raschelgewirke ist so aufgebaut, daß bei einem Flächengewicht von ca. 80 g/m² das Verhältnis von gemitteltem Abstand zweier Kettfäden zum gemittelten Abstand zweier Teilschußfäden 2.7 beträgt. Das Raschelgewirke ist in Längsrichtung starr, weist jedoch eine Querdehnbarkeit von 40 % auf. Die Binden weisen daher gegenüber Verbandsmaterialien auf Basis eines in Quer- und Längsrichtung starren Leinengewebes einen erhöhten Anlegekomfort auf. Die Querdehnbarkeit von 40 % erlaubt es jedoch nicht, den Verband an besonders schwierig zu wickelnden Stellen, wie beispielsweise Ellenbogen oder Ferse, faltenfrei "herüberzuziehen". Die Querdehnbarkeit des Raschelgewirkes, und die damit einhergehende größere Flexibilität gegenüber dem starren Leinengewebe, führt jedoch dazu, daß die Binden auch im ausgehärteten Zustand weniger steife Verbände ergeben. Das bedeutet, daß zum Erzielen eines Stützverbandes gleicher Steifigkeit bei Verwendung von Raschelgewirke des Standes der Technik mehr Material vonnöten ist als bei Einsatz von Leinengewebe.

Eine weitere Möglichkeit, Polyurethanstützverbänden einen erhöhten Anlegekomfort zu geben, besteht in der Verwendung von Glasfaser-Trägermaterialien, so wie sie beispielsweise in der PCT-Patentanmeldung WO 81/00671 offenbart ist. Ein Handelsprodukt, welches Glasfaserbinden in Form einer ebenfalls geraschelten Wirkware mit einer Querdehnbarkeit von 30% enthält, ist Scotchcast R der Firma 3M, USA. Die unbefriedigende Querdehnbarkeit von nur 30 % wird zwar durch eine gute Anschmiegsamkeit der Glasfaser ausgeglichen; das Material härtet darüber hinaus zu Verbänden hoher Festigkeit aus. Von großem Nachteil sind jedoch die mangelnde Röntgentransparenz sowie die Scharfkantigkeit und das schwierige Entfernen solcher Glasfaserverbände.

Es stellt sich somit die Aufgabe, für feuchtigkeitshärtende Polyurethanstützverbände ein Trägermaterial zu entwickeln, welches hohe Röntgentransparenz mit großer Querdehnbarkeit und guter Festigkeit verbindet.

Es wurde nun gefunden, daß überraschenderweise alle diese Eigenschaften in einem Material vereinigt werden können, wenn ein aus Natur- und/oder Kunstfaser hergestelltes, in Längsrichtung starres und in Querrichtung mehr als 100 % dehnbares Raschelgewirke eingesetzt wird, bei welchem bei einem Flächengewicht von 40 bis 150 g/m² das Verhältnis von gemitteltem Abstand zweier Kettfäden zu gemitteltem Abstand zweier Schußfäden kleiner als 1,5 ist.

Gemäß einer besonders bevorzugten Ausführungsform beträgt das Flächengewicht des Raschelgewirkes zwischen standsverhältnis der Kettfäden zu Schußfäden ist ≤ 1 und die Querdehnbarkeit liegt über 200 %. Die erfindungsgemäßen Binden sind daher auch an kritischen Stellen schnell und faltenfrei zu wickeln. Erstaunlicherweise zeigen die erfindungsgemäßen Verbandmaterialien gegenüber jenen des Standes der Technik trotz erhöhter Porosität eine stark erhöhte Festigkeit, und zwar selbst dann, wenn bei den Binden des Standes der Technik das Flächengewicht der Gewirke bis zu 20 % höher ist.

Völlig überraschend ist jedoch die Tatsache, daß die erfindungsgemäß zu verwendenden Gewirke selbst bei Querdehnbarkeit von > 200 % und bei großer Porosität die Festigkeit der starren und relativ dichten Leinengewebe übertreffen. Dies war insofern nicht zu erwarten, da der Stand der Technik offenbarte, daß bei annähernd gleichen Flächengewichten und Imprägnierraten Stützverbände auf Basis von Gewirken in ihrer Steifigkeit den Verbänden auf Basis von Geweben unterlegen sind, sofern die gleichen textilen Basismaterialien eingesetzt werden.

Für die Herstellung der erfindungsgemäß zu verwendenden Gewirke kommen sowohl Naturfasern wie Baumwolle oder Wolle als auch Kunststoffasern, wie z.B. aliphatische oder aromatische Polyamide, Polyester, Polyacrylnitril, Cellulose oder Kohlenstoffasern in Betracht. Auch beliebige Mischungen dieser Materialien sind verwendbar.

Erfindungsgemäß bevorzugt sind Gewirke aus Baumwolle und/oder Polyesterfasern. Die Gewirke können nach den an sich bekannten Wirktechniken hergestellt werden, wobei jedoch auf das erfindungswesentliche Abstandsverhältnis von Kett- zu Schuß (bzw. Teilschuß)-fäden zu achten ist.

Als feuchtigkeitshärtende Isocyanatharze, mit denen die Binden aus Raschelgewirke imprägniert werden, kommen z.B. produkte in Betracht, wie sie in den oben erwähnten Deutschen Auslegeschriften 2 651 089 und 2 357 931 beschrieben sind. Einsetzbar ist jede Art von mindestens zwei NCO-Gruppen aufweisenden, aliphatischen, cycloaliphatischen oder vorzugsweise aromatischen Isocyanaten. Bevorzugt sind die aus der Polyurethanchemie an sich bekannten präpolymeren mit endständigen NCO-Gruppen, also Umsetzungsprodukte von Polyolen (insbesondere Polyester- oder Polyetherpolyolen) mit überschüssigem Polyisocyanat. Bevorzugt als Isocyanatkomponenten sind produkte, die sich aus phosgenierten Formaldehyd-Anilin-Kondensaten ableiten und gegebenenfalls durch Einbau von Harnstoff-, Urethan- oder Carbodiimidgruppen modifiziert wurden. Die Härtungsreaktion mit Wasser, welche durch Tauchen der Binden in Wasser oder durch Exposition an der Luftfeuchtigkeit bewerkstelligt wird, wird bevorzugt durch tertiären Aminstickstoff aufweisende Substanzen beschleunigt. Bevorzugt ist dabei der tertiäre Aminstickstoff in das Präpolymere eingebaut, wie es z.B. in der DAS 2 651 089 beschrieben wird. Gegebenenfalls können die Isocyanatharze auch Additive, z.B. Füllstoffe oder Lichtschutzmittel (z.B. gemäß DE-OS 2 921 163) enthalten.

Die Imprägnierrate, die als das Verhältnis von Harzgewicht zu Bindengewicht definiert ist, beträgt vorzugsweise 50 bis 200 %, besonders bevorzugt 120 bis 180 Um eine vorzeitige Aushärtung der Binden durch eindringende Feuchtigkeit zu verhindern, werden sie in Behältnisse, die gegen Wasserdampf undurchlässig sind, verpackt. Beispiele solcher Verpackungsmaterialien sind siegelbare Kunststoff-Aluminium-Laminate. Bevorzugt werden die Binden zu diesen Behältnissen auch unter Ausschluß von Sauerstoff, also z. B. in einer Stickstoff- oder anderen Inertgasatmosphäre, gelagert, wie es in DE-OS 3 033 659 beschrieben wird.

Weitere bevorzugte Ausführungsformen der Erfindung gehen aus den Unteransprüchen hervor.

Im Versuchsteil wird exemplarisch der Aufbau der erfin-dungsgemäß zu verwendenden Gewirke in vergleichender Weise dem Aufbau der textilen Flächengebilde des Standes der Technik gegenübergestellt. Ebenso werden Festigkeitsmessungen an unter identischen Bedingungen angefertigten und ausgehärteten Probekörpern verglichen.

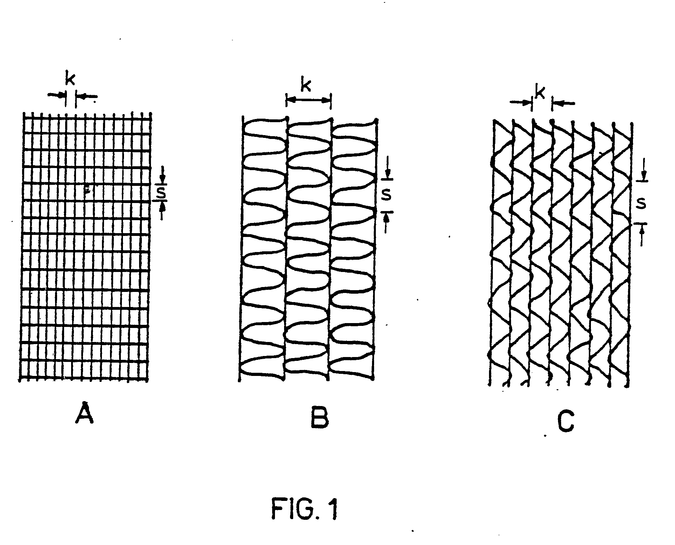

Die verwendeten textilen Trägermaterialien sind schematisch in Fig. 1 dargestellt. A ist das Leinengewebe, B ein Gewirke gemäß Stand der Technik und C ein erfindungsgemäß einzusetzendes Raschelgewirke. Die Materialien sind durch die in der nachstehenden Tabelle 1 angegebenen Maßzahlen charakterisiert.

Tabelle 1

| Gewebe/Gewirke | A | B | C |
|---|---|---|---|
| Flächengewicht ($g/m^2$) | 60 | 80 | 60 |
| Zahl der Kettfäden/cm | 12,1 | 2,5 | 5,8 |
| Zahl der Schußfäden/cm | 6,5 | 6,5 | 4 |
| k (mm) | 0,84 | 4,0 | 1,8 |
| s (mm) | 1,5 | 1,5 | 2,4 |
| k/s | 0,56 | 2,66 | 0,75 |
| Querdehnbarkeit | 0 | 40 | 2 50 |

k   mittlerer Abstand zweier Kettfäden

s   mittlerer Abstand zweier Schußfäden

4

Das Raschelgewirke B besteht aus 60 % Baumwoll- und 40 % Polyesterfasern; C besteht zu 100 % aus Baumwolle.

## Herstellung der Probekörper

Die Trägermaterialien A, B und C werden als Bänder von 10 cm Breite und 3 m Länge mit einem Harz imprägniert, welches aus 100 Gew.-Teilen eines phosgenierten Anilin-Formaldehyd-Kondensationsprodukts mit 30 Gew.-% NCO-Gruppen und einer Viskosität von 200 m Pas bei 25°C und aus 32 Gew.-Teilen eines durch Propoxylierung von Triethanolamin erhaltenen Trihydroxypolyesters (OH-Zahl 146, Viskosität 1200 mPas/25°C) hergestellt wurde. Die Imprägnierrate, definiert als Quotient aus Harzgewicht und Bindengewicht, beträgt jeweils 150 %.

Die Gewebebahnen werden 10 sec. in Wasser von 25°C getaucht, ausgequetscht und zu einem rollenförmigen Probekörper mit einem Innendurchmesser von 4,6 cm gewickelt. Anschließend werden die Binden 24 Stunden bei Raumtemperatur getrocknet.

## Messung der mechanischen Festigkeit

In einem Beugungsmeßgerät (Hersteller Fa. Zwick, BRD; Modell Z 423) wird die Durchbiegung der Probekörper bei einer Last von 50 kp gemessen. Auf die Oberfläche des Probekörpers wird dabei ein keilförmiger Stempel gedrückt, der parallel zur Längsachse des rollenförmigen Probekörpers ausgerichtet ist und dabei eine Auflagenbreite von 31 mm aufweist.

Es werden folgende Durchbiegungen erhalten:

## Tabelle 2

| Probekörper | A | B | C |
|---|---|---|---|
| Durchbiegung (mm) | 4,8 | 9,6 | 3,8 |

Tabelle 2 zeigt, daß der erfindungsgemäße Stützverband jenen aus dem Gewirke des Standes der Technik trotz geringeren Flächengewichts, größerer Querdehnbarkeit und porosität in der Festigkeit um 126 % übertrifft. Auch das starre und relativ dichte Leinengewebe wird um 26 % übertroffen.

## Patentansprüche:

1. Verbandmaterial zur Herstellung von Stützverbänden, bestehend aus einem Raschelgewirke aus Natur- und/oder Kunststoffaser als Trägermaterial, welches mit einem feuchtigkeitshärtenden, Isocyanatgruppen aufweisenden Harz beschichtet und/oder imprägniert ist, dadurh gekennzeichnet, daß das Raschelgewirke ein Flächengewicht von 40 bis 150 $g/m^2$, ein Verhältnis von mittlerem Abstand zweier Kettfäden zu mittlerem Abstand zweier Schußfäden von weniger als 1,5 und eine Querdehnbarkeit von mehr als 100 % aufweist.

2. Verbandmaterial gemäß Anspruch 1, dadurch gekennzeichnet, daß das Raschelgewirke ein Flächengewicht von 50 bis 100 $g/m^2$ aufweist.

3. Verbandmaterial gemäß Anspruch 1 oder 2, dadurch gekennzeichnet, daß das Raschelgewirke aus Baumwolle besteht.

4. Verbandmaterial gemäß Anspruch 1 oder 2, dadurch gekennzeichnet, daß das Raschelgewirke aus Polyesterfaser besteht.

5. Verbandmaterial gemäß Anspruch 1 oder 2, dadurch gekennzeichnet, daß das Raschelgewirke aus einer Mischung von Baumwolle und Polyesterfaser besteht.

6. Verbandmaterial gemäß Anspruch 1 bis 5, dadurch gekennzeichnet, daß das zur Imprägnierung verwendete feuchtigkeitshärtende Harz auf einem Polyisocyanat basiert, welches durch Phosgenierung eines Anilin-Formaldehydkondensats erhalten wurde.

7. Verbandmaterial gemäß Anspruch 1 bis 6, dadurch gekennzeichnet, daß die Imprägnierrate zwischen 120 und 180 % liegt.

8. Verbandmaterial gemäß Anspruch 1 bis 7, dadurch gekennzeichnet, daß das Verhältnis von mittlerem Abstand zweier Kettfäden zu mittlerem Abstand zweier Schußfäden des Raschelgewirkes ≦ 1 ist.

9. Verbandmaterial gemäß Anspruch 8, dadurch gekennzeichnet, daß das Verhältnis zwischen 0,5 und 1 liegt.

10. Verbandmaterial gemäß Anspruch 1 bis 8, dadurch gekennzeichnet, daß die Querdehnbarkeit des Raschelgewirkes größer als 200 % ist.

**Claims**

1. Bandaging material for the production of support bandages consisting of a double rib worked material of natural and/or synthetic fibre as base material, which is coated and/or impregnated with a moisture-curing resin containing isocyanate groups, characterised in that the double rib worked material has a surface weight of from 40 to 150 g/m$^2$, a ratio of the average spacing between two warp threads to the average spacing between two weft threads of less than 1.5 and a transverse extensibility of greater than 100%.

2. Bandaging material according to claim 1, characterised in that the double rib worked material has a surface weight of from 50 to 100 g/m$^2$.

3. Bandaging material according to claim 1 or 2, characterised in that the double rib worked material consists of cotton.

4. Bandaging material according to claim 1 or 2, characterised in that the double rib worked material consists of polyester fibre.

5. Bandaging material according to claim 1 or 2, characterised in that the double rib worked material consists of a mixture of cotton and polyester fibre.

6. Bandaging material according to claim 1 to 5, characterised in that the moisturecuring resin used for impregnation is based on a polyisocyanate which is obtained by phosgenation of an aniline formaldehyde condensation product.

7. Bandaging material according to claim 1 to 6, characterised in that the degree of impregnation.is from 120 to 180%.

8. Bandaging material according to claim 1 to 7, characterised in that the ratio of the average spacing between two warp threads to the average spacing between two weft threads of the double rib worked material is $\leq$ 1.

9. Bandaging material according to claim 8, characterised in that the ratio is from 0.5 to 1.

10. Bandaging material according to claim 1 to 8, characterised in that the transverse extensibility of the double rib worked material is greater than 200%.

**Revendications**

1. Matériau de bandage pour la fabrication de bandages de soutien, consistant an un tricot Rachel en fibres naturelles et/ou synthétiques comme matériau de support, qui est enduit et/ou imprégné avec une résine présentant des groupes isocyanate et durcissant à l'humidité, caractérisé en ce que le tricot Rachal présente un grammage de 40 à 150 g/m$^2$, un rapport d'écartement moyen de deux fils de chaîne envers l'écartement de deux fils de trame inférieur à 1,5 et une extensibilité transversale supérieure à l00%.

2. Matériau de bandage selon la revendication 1, caractérisé en ce que le tricot Rachel présente un grammage de 50 à 100 g/m$^2$.

3. Matériau de bandage selon la revendication 1 ou 2, caractérisé en ce que le tricot Rachel consiste en du coton.

4. Matériau de bandage selon la revendication 1 ou 2, caractéris%51 en ce que le tricot Rachel consiste en de la fibre polyester.

5. Matériau de bandage selon la revendication 1 ou 2, caractérisé en ce que le tricot Rachel consiste en un mélange de coton et de fibre polyester.

6. Matériau de bandage selon les revendications 1 à 5, caractérisé en ce que la résine durcissant à l'humidité, utilisée pour l'imprégnation, est à base d'un polyisocyanate qui a été obtenu par phosgénation d'un condensat aniline-formaldéhyde.

7. Matériau de bandage selon les revendications 1 à 6, caractérisé en ce que les taux d'impregnation se situent entre 120 et 180%.

8. Matériau de bandage selon les revendications 1 à 7, caractérisé en ce que le rapport de l'écartement moyen de deux fils de chaine envers l'écartement moyen de deux fils de trame du tricot Rachel est inférieur ou égal à 1.

9. Matériau de bandage selon la revendication 8, caractérisé en ce que le rapport est compris entre 0,5 et 1.

10. Matériau de bandage selon les revendications 1 à 8, caractérisé en ce que l'extensibilité transversale du tricot Rachel est supérieure à 200%.

FIG. 1